# EUROPEAN PATENT APPLICATION

(11) **EP 2 786 785 A1**
(43) Date of publication of application: **08.10.2014**
(21) Application number: 12853324.7
(22) Date of filing: 22.11.2012
(51) Int. Cl.: A62B 23/06, A61F 5/56

(54) **DEVICE FOR RELIEVING THE SYMPTOMS OF NASAL ALLERGIES**

(30) Priority: 28.11.2011 ES 201131925 P
(71) Applicant: Best Breathe Company, S.L., 03430 Onil, Alicante (ES)
(72) Inventor:
(74) Representative: Isern-Jara, Nuria
(86) International application number: PCT/ES2012/070817
(87) International publication number: WO 2013/079751

(57) **Abstract**

Device for relieving the symptoms of nasal allergies, which comprises a single, one-piece body formed by two tubular elements connected by an intermediate bridge, which elements are provided in order to be inserted into the nasal cavities. The tubular elements have continuous or discontinuous external projections such that at least one free passage is created over the entire height of the tubular element, which allows the evacuation of fluids from the nose.

## Description

### OBJECT OF THE INVENTION

The object of the present Invention Patent application is to register a device for relieving the symptoms of allergies produced by elements that enter through the nasal passages, and specifically to relieve the symptoms of allergies when there is obstruction, or malformations, whether congenital or of any other sort, in the nasal cavities, and more specifically devices with tubular elements which are inserted into the nasal cavities to facilitate breathing.

### BACKGROUND OF THE INVENTION

There are currently many known types of means that make it possible to improve breathing in certain cases wherein users have mucous, malformations of the nose, etc., independently of medical treatment.

Said means may consist of a simple bandage-like element affixed externally over the nose bridge so as to pull at the sides of the nose and open up the nasal cavities. This device has been used by athletes and other people, and the results in terms of opening up the nasal cavities have left much to be desired.

There is, in addition, another device made up of two short tubes that are inserted into the nasal cavities, and which are joined together by means of a bridge, which acts as a joining element between said tubes, and as a stopping element for the maximum penetration of the tubes into the nasal cavities. The inner surface of these short tubes is equipped with a threading worm for coupling corresponding end pieces, which establish the air intake mouth for air passing from the nasal cavities outwards. The drawback of this type of devices is that it comprises three independent elements or parts, two of them corresponding to the short tubes, and the other to the bridge, to which the end pieces that couple to the threaded portions of the tubes must be added, making it a device formed by five parts. This entails, on the one hand, high manufacturing costs for the device, and significant handling requirements when assembling the various parts together.

All of this involves high financial costs, as well as being a nuisance for users, in addition to the possibility that the end pieces could be left inside the nasal cavities when removing the device if the former have not been screwed in properly, thus creating further nuisances and problems for the user.

Another characteristic posed by the known devices is that they have round, ring-shaped external projections to help secure them inside the nasal cavities, with the drawback that said projections constitute an element that retains the liquids and fluids in the nose, hindering their evacuation and causing the air intake to become obstructed, and increasing the drawbacks and nuisances that cleaning said fluids entails.

However, there is no known device that, while carrying out the above functions, is able to improve the health conditions of people with allergies, relieving the common discomforts associated with this type of illnesses.

Therefore, it would be desirable for there to be a device that could efficiently open up the nasal cavities, while avoiding the drawbacks found in the above devices in the state of the art, and improve the health conditions of users suffering from allergies.

### DESCRIPTION OF THE INVENTION

The present invention solves the problems found in the state of the art by means of a device for relieving the symptoms of allergies, which comprises a single, one-piece body, in turn formed by two tubular elements provided in order to be inserted into the nasal cavities and keep them open. The tubular elements are connected to one another by means of an intermediate bridge, which acts as a stopping element to keep the tubular elements from penetrating too far into the nasal cavities.

This single piece provides a number of advantages over the devices found in the state of the art, such as greater structural simplicity and a smaller number of elements, which translates into simpler manufacturing, without the need to couple any parts, and without end pieces that could be left inside the nasal cavities when joined incorrectly.

In addition, on the outer surface of the tubular elements there is a plurality of external projections that facilitate their insertion into the nasal cavities, avoiding scratches or ulcerations inside the same. These projections facilitate the retention of the device inside the nasal cavities and the evacuation of any fluids produced inside of them, creating at least one free passage all along the height of the tubular element, thus keeping them from accumulating and obstructing the tubular elements. For this purpose, said projections can have two preferred forms of embodiment, one of which is through partial projections that allow for free areas or evacuation channels between them to facilitate the outflow or expulsion of nasal fluids. In another form of embodiment, the projection is constituted by a continuous element, wherein its initial and final ends do not match up, and each of which is located near the ends or edges of the tubular elements, preferably with a helical shape.

Furthermore, there is also the possibility of arranging, inside at least one of the tubular elements, at least one ridge, preferably with a helical shape, which projects inwards, making it more difficult for certain particles, such as dust, sand, or pollen, to enter the nasal cavities, relieving and reducing allergy symptoms. Therefore, these ridges serve to substitute the hairs inside the nasal cavities, which are unable to carry out their protective function once the tubular elements have been inserted. Moreover, because the ridges will be moistened by the effects of the nose itself, any particles that might enter the nose will encounter a further obstacle to making their way inside the respiratory tract.

In another preferred form of embodiment, the ridges may be defined in partial sections or segments inside the tubular elements.

Other characteristics and advantages of the device for relieving symptoms of nasal allergies, object of the present invention, will become clear in light of the description of the preferred, though non-exclusive, embodiments, which, by way of a non-limiting example, are illustrated in the accompanying drawings, wherein:

### BRIEF DESCRIPTION OF THE DRAWINGS

Figure 1.- Is a perspective view of a device for relieving symptoms of nasal allergies, in accordance with the present invention, in a state prior to use.
Figure 2.- Is a cross-section view showing a specific embodiment of the device with a continuous internal ridge arranged helically inside both of the tubular elements.
Figure 3.- Is a cross-section view showing an alternative embodiment of the device, with separate and staggered ridges, arranged helically inside the tubular elements.
Figure 4.- Is a perspective view of a third embodiment of the device of the invention.
Figure 5.- Is a perspective view of a fourth embodiment of the device of the invention.

### DESCRIPTION OF PREFERRED EMBODIMENTS

As is shown in the attached figures, one can see two embodiments of a device for relieving symptoms of nasal allergies to be treated. More specifically, the device comprises a single, one-piece body (1), preferably made of a flexible material, and which in turn is formed by two tubular elements (2) provided in order to be inserted into a user's nasal cavities and keep them open. As may be observed in the figures, on the outer surface of the tubular elements (2) there is a plurality of external projections (3) that facilitate their insertion into the nasal cavities, avoiding scratches or ulcerations inside the same. The figures show a specific embodiment wherein the projections (3) have a rounded shape, and partial sections of a ring-like configuration, with free spaces between said sections which allow the evacuation of nasal fluids. In another alternative embodiment, which has not be represented, the internal projections are constituted by at least one continuous and round helical projection, such that its ends match up with opposite edges of the tubular element, thus facilitating the aforementioned evacuation.

The tubular elements (2) are connected to one another by means of an intermediate bridge (4), which acts as a stop, keeping the tubular elements (2) from penetrating too far inside of the nasal cavities.

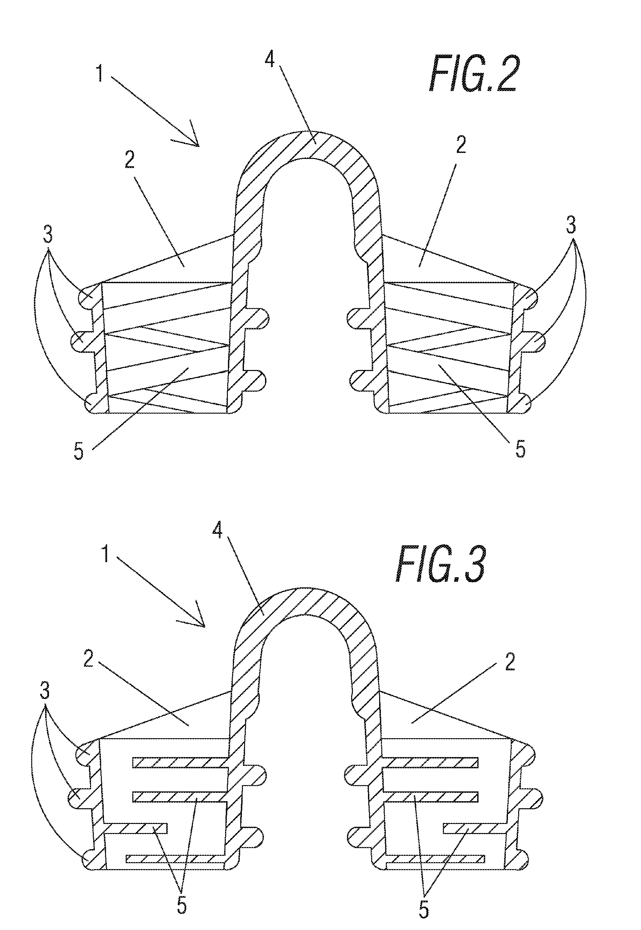

Figure 2 shows a specific embodiment of the invention in which inside both of the tubular elements (2) there is a continuous ridge (5) arranged helically all along said tubular element (2), making it more difficult for particles such as dust, sand or pollen to enter the nasal cavities, which contributes to reducing the effects of allergies.

Figure 3 shows an alternative embodiment to the preceding one, wherein the parts in common have the same numerical references as in the embodiment in figure 2, in which inside both of the tubular elements (2) there is a plurality of short ridges (5) arranged helically all along the tubular element (2), achieving the same effect as the preceding embodiment.

Preferably, the device for relieving symptoms of nasal allergies (1) is made of medical-grade silicone, although they may be made of another flexible material.

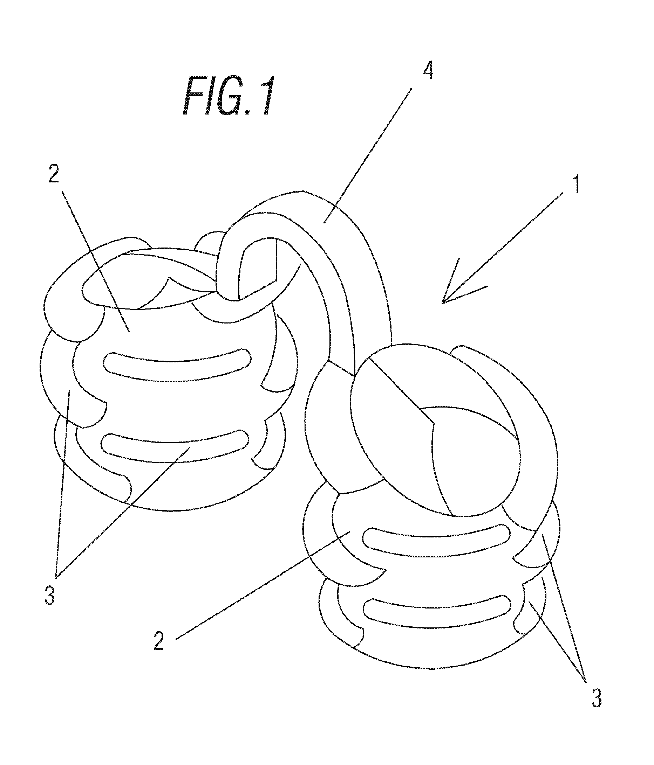

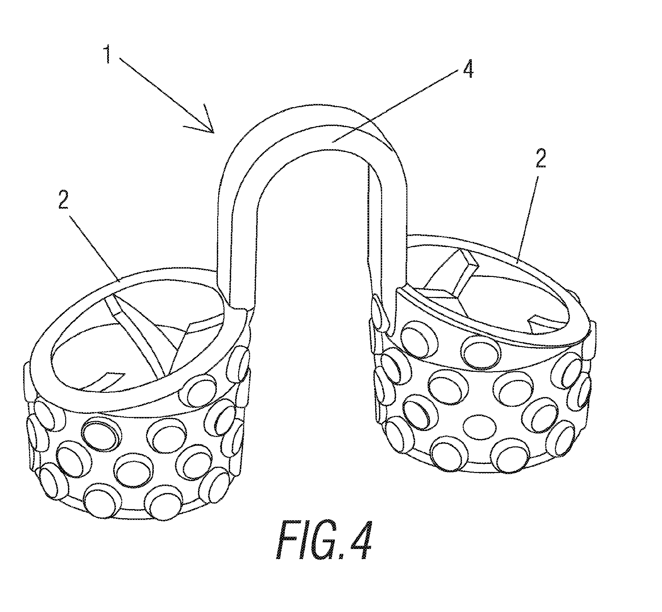

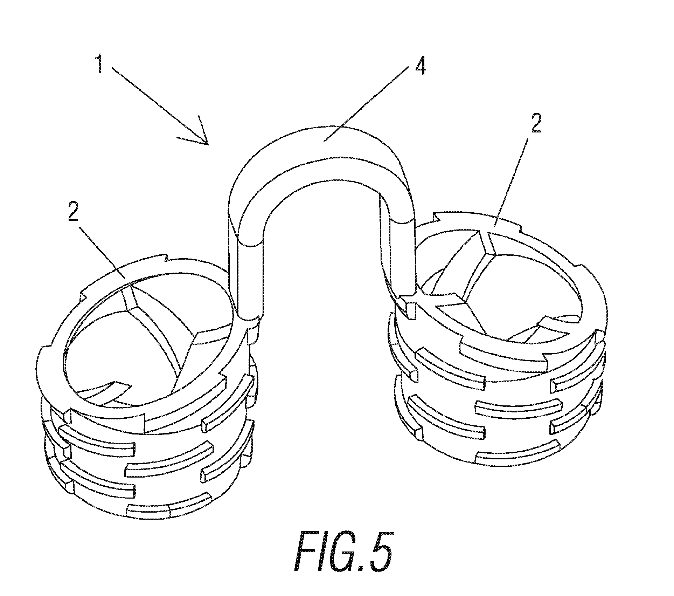

Figures 4 and 5 show two alternative embodiments to the ones described above, wherein the parts in common have the same numerical references as in the embodiment in figure 1. The external projections in figure 4 take the form of circular sections, whereas in figure 5 the external projections take the form of essentially straight segments in rows, which do not line up with one another.

The details, shapes, dimensions and other accessory elements, as well as the materials used to manufacture the device for relieving symptoms of nasal allergies of the invention, may be suitably substituted for others which are technically equivalent, and do not diverge from the essential nature of the invention, nor the scope defined by the claims included below.

## Claims

1. Device for relieving the symptoms of nasal allergies, which comprises a one-piece body (1), which in turn comprises two tubular elements (2) provided in order to be inserted into the nasal cavities and keep them open, said tubular elements (2) being connected to one another by means of an intermediate bridge (4), **characterized in that** the surface of the tubular elements comprises at least one external projection (3) which is continuous, wherein its initial and final ends do not match up and each end of the ridge is located near the opposite edges of the tubular elements (2), or discontinuous, such that at least one free passage is created all along the height of the tubular element to allow the evacuation of nasal fluids.

2. Device for relieving the symptoms of nasal allergies, according to claim 1, **characterized in that** inside the tubular elements at least one ridge (5) for retaining allergy-causing particles is provided.

3. Device for relieving the symptoms of nasal allergies, according to claim 1, **characterized in that** the tubular elements (2) have a plurality of external projections (3) with any sort of discontinuous configuration all along the external face of the tubular elements (2), leaving free evacuation spaces or channels between them, thus keeping liquids and secretions originating inside the nasal cavities from being retained in the device and obstructing the air flow.

4. Device for relieving the symptoms of nasal allergies, according to claim 1, **characterized in that** the continuous shape of the external projections (3) of the tubular elements (2) takes the form of a helical structure.

5. Device for relieving the symptoms of nasal allergies, according to claim 2, **characterized in that** ridges (5) are arranged helically on the inner wall of the tubular elements (2).

6. Device for relieving the symptoms of nasal allergies, according to claim 2, **characterized in that** ridges (5) are arranged over the entire length of the tubular element (2).

7. Device for relieving the symptoms of nasal allergies, according to claim 2, **characterized in that** ridges (5) are arranged in partial segments or sections inside the tubular elements (2).
